# EUROPÄISCHE PATENTSCHRIFT

(11) **EP 2 300 080 B1**
(45) Veröffentlichungstag und Bekanntmachung des Hinweises auf die Patenterteilung: **13.05.2020**
(21) Anmeldenummer: 09753399.6
(22) Anmeldetag: 19.05.2009
(51) Int. Cl.: A61M 5/24, A61M 5/32, A61M 5/46, A61M 5/34

(54) **ABMISCHVORRICHTUNG FÜR EINE ZWEIKAMMERAMPULLE**
MIXING DEVICE FOR A TWO-CHAMBER AMPOULE
DISPOSITIF DE MÉLANGE POUR UNE AMPOULE À DEUX CHAMBRES

(30) Priorität: 24.05.2008 DE 102008025002
(43) Veröffentlichungstag der Anmeldung: 30.03.2011
(73) Patentinhaber: Tecpharma Licensing AG, 3401 Burgdorf (CH)
(72) Erfinder: MORI, Kevin, 3415 Hasle bei Burgdorf (CH); KAENEL, Céline, 8032 Zürich (CH); DRUNK, Annette, 3007 Bern (CH); HIRSCHEL, Jürg, 5000 Aarau (CH); MOSER, Ulrich, 3412 Heimiswil (CH); TSCHIRREN, Markus, 3422 Kirchberg (CH); WYMANN, Martin, 3097 Liebefeld (CH); TUCKWELL, Jonathan, D., Cambridge, MA 02142 (US); HARRIS, Scott, San Diego, CA 92121 (US); RYAN, Patrick, J., Cambridge, MA 02142 (US); PIDGEON, Andrew, D., Cambridge, MA 02142 (US); MANN, Richard, Cambridge, MA 02142 (US)
(86) Internationale Anmeldenummer: PCT/CH2009/000165
(87) Internationale Veröffentlichungsnummer: WO 2009/143640

(56) Entgegenhaltungen:
- EP-A1- 1 790 366
- DE-A1-102004 055 298
- DE-U1- 9 201 074

## Beschreibung

Die Erfindung betrifft eine Vorrichtung zur Verabreichung eines flüssigen Produkts, wie z.B. eines Medikaments, in den Körper eines Patienten. Die Vorrichtung dient ferner dazu, ein in einer Ampulle, insbesondere Zweikammerampulle enthaltenes Produkt abzumischen.

Aus der DE 103 40 585 A1 ist eine Zweikammerampulle mit entsprechender Vorrichtung zur Verabreichung und zum Abmischen bekannt. Zum Abmischen wird eine Zweikammerampulle aufnehmende Ampullenhalterung in ein Gehäuse der Injektionsvorrichtung bewegt. Dabei kann der Verwender den hinteren Gehäuseabschnitt und den vorderen Abschnitt der Ampullenhalterung greifen und gegeneinander verdrehen, wodurch ein Abmischvorgang stattfindet. Aus der EP1790366 A1 ist eine Injektionsvorrichtung bekannt, welche eine Kolbenstange mit zwei federn umfasst, von denen die distale Feder eine Mischung von Bestandteilen zu einem Produkt in der Zweikammerampulle bewirkt, indem sie eine proximale Kolbenstange der Zweikammerampulle linear gegen einen distalen Kolben der Zweikammerampulle bewegt.

Es ist eine Aufgabe der Erfindung, eine Vorrichtung zur Verabreichung eines Medikaments bereitzustellen, welche intuitiv bedienbar ist.

Die Aufgabe wird gelöst mit den Merkmalen des unabhängigen Anspruchs. Vorteilhafte Weiterentwicklungen ergeben sich aus den abhängigen Ansprüchen und der Beschreibung einschließlich der Figuren.

Der Gegenstand der Erfindung ist durch den unabhängigen Anspruch 1 definiert.

Die Erfindung geht aus von einer Vorrichtung zum Injizieren und/oder Mischen eines zu injizierenden Produkts. Das Produkt kann z.B. ein aus zwei oder mehreren Bestandteilen gemischtes oder zu mischendes Produkt sein. Das zu mischende Produkt kann aus mehreren Bestandteilen bestehen, die z.B. in flüssiger oder fester, trockener, insbesondere partikel- oder pulverförmiger Phase vorliegen. Es sind somit Injektionsgeräte bevorzugt mit einer Zweikammerampulle, bei der das Medikament vor einer Injektion erst aus zwei Bestandteilen zusammengemischt bzw. abgemischt werden muss. Zweikammerampullen weisen in der Regel eine erste Kammer für den ersten Produktbestandteil und eine zweite Kammer für einen zweiten Produktbestandteil auf, wobei im Ausgangszustand die beiden Bestandteile z.B. durch eine Membran oder einen Kolben voneinander getrennt sind. Die getrennte Lagerung der Bestandteile kann die Lagerfähigkeit :des Medikaments erhöhen, da das abgemischte Medikament nur von relativ kurzer Haltbarkeit ist. Beim Abmischen werden die Bestandteile zusammengeführt. Das Abmischen kann z.B. durch das Einsetzen der Zweikammerampulle in eine erfindungsgemäße Vorrichtung geschehen.

Die Vorrichtung umfasst eine Aufnahme für das oder Bestandteile des zu verabreichenden Produkts. Die Aufnahme kann z.B. hülsenförmig sein, wobei das Produkt innerhalb des hülsenförmigen Abschnitts angeordnet sein kann. Die Aufnahme dient zur Aufnahme Zweikammerampulle. Im Folgenden wird die Erfindung mit einer Zweikammerampulle stellvertretend für alle in Frage kommenden Produktbehältnisse beschrieben.

Die Zweikammerampulle kann erfindungsgemäß vorteilhaft mit einer Ampullenhalterung an einer Injektionsvorrichtung angeordnet werden, wobei die Ampullenhalterung eine Ampullenaufnahme und ein Befestigungsglied aufweist. Die Ampulle kann einen Verschluss aufweisen, der das distale Ende der Ampulle z.B. für ein Stechmittel durchstechbar verschließt. Der Verschluss kann eine radiale Abragung aufweisen, die sich beispielsweise radial über den zylindrischen Durchmesser des die Kolben der Ampulle lagernden Teils erstreckt oder sogar den größten Durchmesser der gesamten Ampulle aufweist. Die radiale Abragung des Verschlusses kann zwischen Ampullenhalterung und Befestigungsglied angeordnet, insbesondere eingefasst sein. Hierdurch wird es ermöglicht, die Ampulle axial zu fixieren, d.h. für eine Bewegung in distale und proximale Richtung. Ein gegebenenfalls auftretendes axiales Spiel des Produktbehältnisses kann, wie weiter unten beschrieben wird, verringert oder sogar beseitigt werden.

Die Ampullenaufnahme kann z.B. hülsenförmig sein mit einem Innendurchmesser, der in etwa dem Außendurchmesser des zylindrischen Teils der Ampulle entspricht, so dass die Ampulle seitlich von der Ampullenaufnahme geführt wird. Die Ampullenaufnahme kann z.B. ein Gewinde aufweisen, insbesondere ein Außengewinde, das in ein entsprechendes Gegengewinde einer Injektionsvorrichtung eingreifen kann, so dass die Ampullenaufnahme axial in das Gehäuse verschiebbar ist, insbesondere mit einer kombinierten Drehbewegung. Statt des Gewindes können auch eine Axialführung oder andere Komponenten vorgesehen sein, solange sie die axiale Bewegung der Ampullenaufnahme in die Injektionsvorrichtung erlauben.

Das Befestigungsglied kann an der Ampullenaufnahme befestigt oder befestigbar sein. Z.B. kann das Befestigungsglied einteilig mit der Ampullenaufnahme gebildet sein. Bevorzugt sind die Ampullenaufnahme und das Befestigungsglied separate Teile, die zusammen die Ampullenhalterung bilden.

Das Befestigungsglied kann eine Abragung sein oder aufweisen, welche an eine Stirnseite des Verschlusses der Ampulle, insbesondere an die distale Stirnseite, angreift. Z.B. kann die Abragung ein Schnapper oder ein Nocken oder dergleichen sein, wobei sich die Abragung z.B. von außen radial nach innen erstrecken kann.

Die Ampulle kann mit der proximalen Seite des Verschlusses an der Ampullenaufnahme anliegen, wie z.B. an der Stirnseite der Ampullenaufnahme oder an einer an der Ampullenaufnahme von einem inneren Umfang radial nach innen gerichteten Abragung. Bevorzugt weist die Ampullenaufnahme im Bereich, dort wo die Stirnseite des Verschlusses zur axialen Anlage kommen soll, einen Bereich mit einem verringerten Innendurchmesser auf, der kleiner ist als der Außendurchmesser des Verschlusses und bevorzugt größer ist als der zylindrische Teil des Gehäuses. Insbesondere kann eine Stirnfläche der Ampullenaufnahme, wie z.B. das distale Ende, den Anschlag für den Verschluss, insbesondere dessen proximales Ende bieten.

Es ist bevorzugt, dass die Ampulle mit ihrem proximalen Ende zuerst, d.h. mit ihrem zylindrischen Gehäuseteil voran, z.B. durch die proximale Stirnseite der Ampullenaufnahme in die Ampullenaufnahme einführbar oder eingeführt ist. Das Befestigungsglied kann z.B. beim Einführen der Ampulle ausgelenkt werden und in der vollständig eingeführten Position zurückschnappen, um die Ampulle axial in zumindest eine Richtung zu fixieren.

In bevorzugten Ausführungen kann das Befestigungsglied formschlüssig mit der Ampullenaufnahme verbunden werden, insbesondere dreh- und axialfest relativ zur Ampullenaufnahme. Beispielsweise können die Ampullenaufnahme und das Befestigungsglied ineinandergreifende Elemente aufweisen, die z.B. wenn das Befestigungsglied vollständig auf die Ampullenaufnahme aufgesetzt ist, ineinander greifen oder einrasten.

In vorteilhaften Weiterbildungen kann zumindest eines aus Ampullenaufnahme und Befestigungsglied wenigstens eine gegen den Verschluss gerichtete Abragung, wie z.B. einen Nocken, aufweisen. Der wenigstens eine Nocken kann sich in Längsrichtung der Ampulle oder der Ampullenhalterung erstrecken, insbesondere in distale oder proximale Richtung. Bevorzugt liegt der Verschluss, insbesondere mit seiner Stirnseite, an den mindestens einen Nocken an. Durch die durch den Nocken hervorgerufene punktuelle Auflage für den Verschluss kann ein Spiel in der axialen Einfassung des Verschlusses noch besser verringert werden. Die mindestens eine Abragung kann starr oder federnd, insbesondere in Axialrichtung federnd, an der Ampullenaufnahme und/oder dem Befestigungsglied angeordnet sein.

Die mindestens eine Abragung, insbesondere Nocken, kann an der Ampullenaufnahme, nämlich dort, wo das vollständig eingeführte Produktbehältnis in axiale Anlage kommt, angeordnet sein, insbesondere an der Stirnfläche der Ampullenaufnahme. Alternativ oder zusätzlich kann die wenigstens eine Abragung an einer radial nach innen gerichteten Abragung des Befestigungsglieds gebildet sein. Insbesondere dort, wo das Befestigungsglied die Stirnseite des Verschlusses umgreift.

Die Ampullenhalterung, insbesondere die Ampullenaufnahme und/oder das Befestigungsglied, kann Mittel aufweisen, die die Befestigung einer Nadeleinheit an der Ampullenhalterung ermöglichen. Beispielsweise kann die Nadeleinheit aufgesteckt, wie z.B. mit einer konischen Fläche, oder aufgeschraubt, wie z.B. mit einem Gewinde, werden. Bei einer an der Ampulle angeordneten Nadeleinheit kann diese z.B. mit einem Befestigungsabschnitt an der Ampullenhalterung befestigt sein. Bei einer vollständig befestigten Nadeleinheit kann ein Stechmittel, wie z.B. ein Fortsatz oder eine Nadel, das Septum durchstechen und eine Fluidverbindung zwischen einer distalen Nadelspitze der Nadeleinheit und dem Produktbehältnis herstellen. Bevorzugt wird die Nadeleinheit an dem Befestigungsglied festgeschraubt, das z.B. ein Innengewinde aufweist, in das ein Außengewinde der Nadeleinheit eingreifen kann.

Die Ampullenaufnahme kann Mittel, insbesondere ein Gewinde aufweisen, mit den sie an einer Injektionsvorrichtung befestigbar und in befestigtem Zustand mit einer Axialbewegung, insbesondere kombiniert mit einer Drehbewegung, in die Injektionsvorrichtung bewegbar ist. Beispielsweise kann die Ampullenaufnahme ein oder mehrere Rastelemente aufweisen, die bei der Bewegung des Ampullenhalters in die Injektionsvorrichtung an bestimmten Positionen ein akustisches und/oder taktiles Signal erzeugen, indem z.B. das Rastelement über ein Gegenelement rastet.

Die Injektionsvorrichtung weist ein Gehäuse auf, das z.B. eine Mechanik zum Abmischen und/oder Ausschütten des Produkts aufweisen kann. Das Gehäuse ist vorteilhaft hülsenförmig. Das Gehäuse braucht nicht zwangsläufig eine Hülse sein, die stets die Außenseite der Vorrichtung bildet. Vielmehr kann das Gehäuse von einem anderen Element zumindest teilweise überdeckt werden. Die Ampullenaufnahme und das Gehäuse sind in einem Eingriff. Insbesondere kann der Eingriff dergestalt sein, dass das Gehäuse mit einer Axialbewegung, d.h. z.B. mit einer reinen Axialbewegung oder in Kombination mit einer Drehbewegung, in das Gehäuse bewegbar ist. Z.B. können die Aufnahme und das Gehäuse in einem Gewindeeingriff sein. Hierzu können das Gehäuse ein Innengewinde und die Aufnahme ein Außengewinde aufweisen, wobei die Gewinde entsprechend ineinander greifen können.

Das Gehäuse kann an seinem äußeren Umfang eben oder glatt sein, insbesondere kein Gewinde aufweisen. Durch Verdrehung von Aufnahme und Gehäuse relativ zueinander kann sich das Gehäuse über die Aufnahme axial verschieben bzw. sich die Aufnahme axial in das Gehäuse verschieben. Diese Verschiebebewegung kann z.B. zur Freigabe eines Betätigungselements, zum Abmischen des Ampulleninhalts und/oder zum Primen der Ampulle dienen.

Die Vorrichtung hat eine Hülse, die das Gehäuse zumindest teilweise umgeben kann. Daher kann diese Hülse auch als äußere Hülse bezeichnet werden. Die äußere Hülse ist dreh- und axialfest mit der Ampullenhalterung, insbesondere mit der Ampullenaufnahme oder dem Befestigungsglied, dreh- und axialfest verbunden, insbesondere als separates Teil oder einteilig. Die äußere Hülse kann die Ampullenaufnahme an ihrem Umfang zumindest teilweise vorzugsweise zum größten Teil oder vollständig umgeben. Zwischen der äußeren Hülse und der Ampullenaufnahme ist ein Ringspalt gebildet. Erfindungsgemäß befindet sich das Gehäuse im Ausgangszustand, d.h. bevor beispielsweise ein Abmischen oder Primen stattgefunden hat, bereits mit einem Teil, insbesondere seinem distalen Ende in dem Ringspalt. Wenn Ampullenhalterung und Gehäuse ineinandergeschoben werden, kann sich das Gehäuse noch tiefer in den Ringspalt in Richtung distal schieben.

Die äußere Hülse kann sich beispielsweise in proximale Richtung bis über das proximale Ende der Ampulle und/oder das proximale Ende der Ampullenhalterung erstrecken. In distale Richtung kann sich die äußere Hülse beispielsweise bis zum distalen Ende der Ampullenhalterung oder Ampullenaufnahme oder bis zu dem Befestigungsglied erstrecken. Die sich über eine weite Länge der Vorrichtung erstreckende äußere Hülse ermöglicht es dem Verwender, die Vorrichtung fest mit einer Hand, wie z.B. der linken Hand, zu greifen. Die äußere Oberfläche kann z.B. um die Greifbarkeit zu verbessern, eine Oberflächenstruktur, wie z.B. Noppen oder Rippen, oder eine Beschichtung aus einem Material aufweisen, welches einen höheren Reibungskoeffizienten aufweist als das darunter liegende Grundmaterial der äußeren Hülse.

Das Gehäuse kann ebenfalls einen Greifabschnitt aufweisen, der z.B. mit den Fingern der rechten Hand oder ganzen rechten Hand greifbar ist. Der Greifabschnitt kann von der Oberflächenbeschaffenheit so aufgebaut sein wie die äußere Hülse. Bevorzugt ist der Greifabschnitt am proximalen Ende des Gehäuses angeordnet. Der Greifabschnitt kann eine an seinem äußeren Umfang angeordnete Abragung aufweisen, die insbesondere flügelförmig ausgestaltet sein kann und sich mit ihrer schmalen Seite in Axialrichtung erstrecken kann. Die Flügel können plattenförmig und nach außen hin abgerundet sein. Die Flügel können sich radial weiter nach außen erstrecken als die äußere Hülse. Der Vorteil des am proximalen Ende des Gehäuses gebildeten Greifabschnitts ist es, dass auf einfache Weise ein relativ hohes Drehmoment erzeugt werden kann, wodurch das Gehäuse in die äußere Hülse schraubbar ist. Durch die Anordnung von Greifabschnitt und äußerer Hülse kann der Verwender die Injektionsvorrichtung intuitiv bedienen.

Die Vorrichtung kann eine Kolbenstange aufweisen, die von dem Gehäuse aufgenommen ist, die zumindest bei einem Teil der Verschiebung des Gehäuses in die äußere Hülse relativ zum Gehäuse axial bewegbar ist, wobei insbesondere die Kolbenstange proximal aus dem Gehäuse hervortritt. Die Kolbenstange kann z.B. über deren proximales Ende in die Ampulle eingreifen und auf den Kolben wirken, d.h. den Kolben verschieben. Das Gehäuse kann einen Abschnitt aufweisen, welcher die Kolbenstange lagert, insbesondere axial bewegbar und/oder drehfest führt.

Z.B. kann das Gehäuse von einem Ausgangszustand vollständig in das Gehäuse eingeschraubt werden, wobei die Vorrichtung mehrere Sequenzen ausführen kann. Beispielsweise kann ausgehend vom Ausgangszustand eine Vorbereitungssequenz ausgeführt werden, bei der die Kolbenstange oder ein Betätigungselement der Kolbenstange proximal aus dem proximalen Ende des Gehäuses hervortritt. Im Ausgangszustand kann die Kolbenstange bzw. das Betätigungselement insbesondere vollständig unter dem proximalen Ende, wie z.B. dem Greifabschnitt liegen oder von dem Greifabschnitt umgeben sein. In der Vorbereitungssequenz kann die Kolbenstange relativ zu der Ampulle axial fest und zum Gehäuse axial bewegbar sein. Die Kolbenstange kann einen sich über einen Teil ihrer Länge erstreckenden Eingriffsabschnitt aufweisen, in den das Gehäuse oder ein gehäusefestes Element eingreift und insbesondere dort eine begrenzte axiale Verschiebbarkeit der Kolbenstange relativ zu dem Gehäuse zulässt. Bevorzugt bestimmt die Länge des Eingriffabschnitts, wie weit die Kolbenstange proximal aus dem Gehäuse hervortritt. Die Vorbereitungssequenz ist insbesondere dann beendet, wenn die Kolbenstange nicht mehr weiter aus dem Gehäuse hervortreten kann. Kolbenstange und Gehäuse können am Ende der Vorbereitungssequenz und für eine sich nun anschließende Abmischsequenz relativ zueinander axial fest sein, zumindest in eine Richtung, so dass bei einer weiteren Drehung die Kolbenstange vom Gehäuse mitgenommen wird und dabei den Kolben in der Ampulle für einen Abmischvorgang verschiebt. Der Kolben in der Ampulle kann beim Abmischvorgang relativ zu dem anderen Kolben in der Ampulle verschoben werden.

An den Abmischvorgang kann sich nun ein Primevorgang anschließen, der durch fortgesetztes Zusammenschrauben eingeleitet wird, wobei sich beide Kolben in der Zweikammerampulle aneinander anstoßend verschieben. Die Kolbenstange ist dabei relativ zum Gehäuse axial fest zumindest in eine Richtung. Am Ende des Primevorgangs kann ein Axialanschlag und/oder ein Drehanschlag vorgesehen sein, der eine weitere Bewegung des Gehäuses relativ zu dem Ampullenhalter verhindert. Der oder die Anschläge können am proximalen Ende der äußeren Hülse oder am distalen Ende des Ringspalts angeordnet sein. Entsprechend kann das Gehäuse einen entsprechenden Gegenanschlag aufweisen, z.B. im Bereich des Greifabschnitts oder am distalen Ende. Die Anschläge können als Axialanschläge oder als in Umfangsrichtung wirkende Anschläge, nämlich Drehanschläge ausgeführt sein, wobei Drehanschläge gegenüber axial wirkenden Anschlägen den Vorteil haben, dass eine geringere Belastung auftritt, wenn eine Axialbewegung z.B. mit einem Gewinde erzeugt wird. Nach dem Primevorgang ist die Vorrichtung bereit für eine Ausschüttung, die durch Drücken der Kolbenstange in distale Richtung relativ zum Gehäuse erreicht wird. Dabei wird das in der Ampulle enthaltene Produkt ausgeschüttet.

In bevorzugten Ausführungen kann die Aufnahme ein Rastelement aufweisen, das in mindestens einer Position des Gehäuses in Bezug auf die Aufnahme mit mindestens einem Rastgegenglied, insbesondere Rastelement wechselwirkt. Das mindestens eine Rastgegenglied kann am inneren Umfang des Gehäuses angeordnet sein. Beispielsweise kann ein Rastgegenglied so angeordnet sein, dass es am Ende einer der oben genannten Sequenzen mit dem Rastglied wechselwirkt. Beispielsweise kann ein Rastgegenglied für das Ende der Abmischsequenz und ein Rastgegenglied für das Ende der Primesequenz vorgesehen sein. Bei der Wechselwirkung kann ein akustisches und/oder taktiles Signal erzeugt werden, das dem Verwender das Ende der jeweiligen Sequenz anzeigen kann. Das Rastgegenglied kann z.B. eine Abragung oder eine Vertiefung sein. Das Rastglied ist bevorzugt ein federnd an der Ampullenaufnahme gebildeter Nocken. Bei mehreren Rastgegengliedern können diese auf axial unterschiedlichen Positionen und/oder Drehpositionen angeordnet sein.

In bevorzugten Ausführungen kann das Gehäuse mindestens einen Informationsabschnitt aufweisen, der bei der Bewegung des Gehäuses in die äußere Hülse nicht erblickbar ist, z.B. unter der äußeren Hülse verschwindet oder z.B. durch ein in der äußeren Hülse gebildetes Fenster erblickbar ist. Beispielsweise können für eine oder mehrere der auszuführenden Sequenzen unterschiedliche Informationsabschnitte vorhanden sein. Jeder Informationsabschnitt kann z.B. mindestens eines aus folgenden aufweisen: unterschiedliche Farben, unterschiedliche Ziffern oder Zeichen. Beispielsweise kann in einem Fenster die Farbe rot erscheinen, solange die Vorrichtung noch nicht bereit ist für eine Produktausschüttung. Erst wenn die Vorrichtung bereit ist für eine Produktausschüttung kann beispielsweise die Farbe grün im Fenster erscheinen. Alternativ kann für die Primesequenz ein beispielsweise gelber Abschnitt vorgesehen sein, der während der Primsequenz im Fenster erscheint.

In einem anderen Beispiel kann des Gehäuse einen ersten, einen zweiten und einen dritten Informationsabschnitt aufweisen, wobei alle Informationsabschnitte am Gehäuse erblickbar sind, wobei die Informationsabschnitte je nach Sequenz nacheinander unter die äußere Hülse verschoben werden.

An das distale Ende der Injektionsvorrichtung ist eine Nadeleinheit anbringbar. Vorzugsweise ist die Nadeleinheit an der Ampullenhalterung, insbesondere an deren Befestigungsglied, befestigbar, z.B. mit einer Steckverbindung oder einem Gewinde.

Die Erfindung wurde anhand mehrerer Ausführungsbeispiele beschrieben. Im Folgenden wird eine Ausführung der Erfindung anhand von Figuren beschrieben. Die hierbei offenbarten Merkmale bilden je einzeln und in Kombination die Erfindung vorteilhaft weiter. Es zeigen:
- Figuren 1-4: Teile einer Nadeleinheit,
- Figuren 5 und 6: die Einzelteile einer Ampullenhalterung und eine bevorzugte Ausführung einer Nadeleinheit,
- Figur 7: eine zusammengesetzte Ampullenhalterung zusammen mit einer darin aufgenommenen Ampulle,
- Figur 8: die Ampullenhalterung aus Figur 7 mit einer daran und einer an der Ampulle angeordneten Nadeleinheit,
- Figur 9: eine bevorzugte Ausführung eines Befestigungsabschnitts der Nadeleinheit, und
- Figur 10: eine bevorzugte Ausführung einer Verabreichungsvorrichtung.

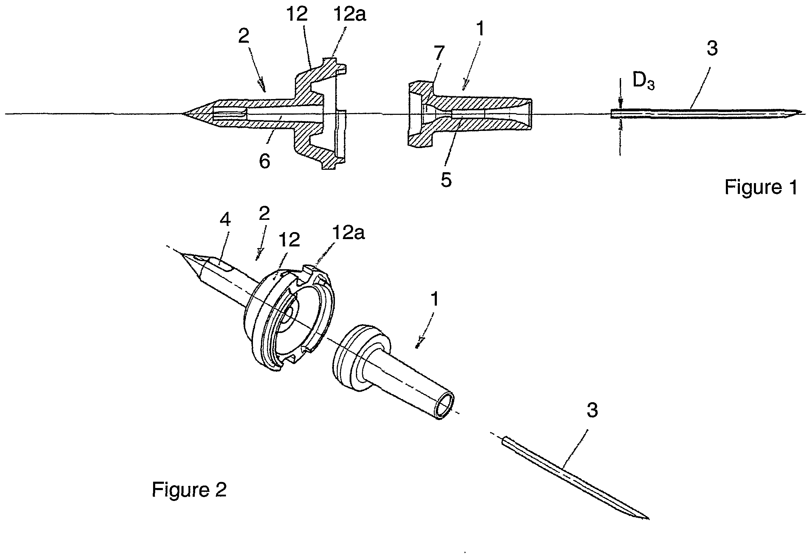

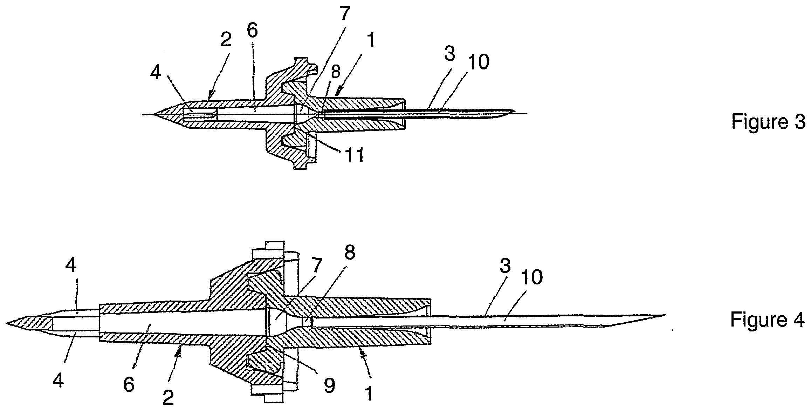

Bezugnehmend auf die Figuren 1-4 werden mehrere Teile einer Nadeleinheit gezeigt, die einen Fluidkanal 6, 7, 8, 10, der auch als Fluidführungskanal bezeichnet werden kann, bilden. Die den Fluidkanal 6, 7, 8, 10 bildenden Teile sind ein Fortsatz 2, der auch als Stechmittel bezeichnet werden kann, ein Nadelträger 1, der auch als Kanülenträger bezeichnet werden kann, und eine Injektionsnadel 3, die auch als Kanülenrohr bezeichnet werden kann.

Die hohlzylindrische Injektionsnadel 3 weist bevorzugt einen konstanten Außendurchmesser D₃ sowie einen konstanten Innendurchmesser auf. An ihrem distalen Ende befindet sich eine Schneidspitze, die im gezeigten Schnitt asymmetrisch ist. Die Injektionsnadel 3 dient zum Einstechen in die Haut eines Patienten und ist mit ihrem proximalen Ende an dem Nadelträger 1 befestigt. Die hierin gezeigte Befestigung besteht darin, dass ein Teil der Injektionsnadel in einer hohlzylindrischen Bohrung 5 aufgenommen ist, deren Innendurchmesser in etwa dem Außendurchmesser der Nadel 3 entspricht. Grundsätzlich könnte die Nadel 3 in dem hohlzylindrischen Abschnitt des Nadelträgers 1 eingepresst oder eingegossen sein, wie z.B. bei der Spritzgussherstellung des Nadelträgers 1. Bevorzugt ist die Nadel in den Nadelträger 1 eingeklebt. Um Spannungsspitzen bei auf die Nadel 3 ausgeübten Querkräften zu verhindern, schließt sich an den Abschnitt 5 ein sich aufweitender, insbesondere trompetenförmig aufweitender Bereich an, der wie hier gezeigt ist, zum distalen Ende des Nadelträgers 1 reicht. Dieser Bereich kann alternativ oder zusätzlich auch dazu dienen, Klebstoff für die Klebeverbindung zwischen dem Nadelträger 1 und der Nadel 3 aufzunehmen. Bevorzugt wird somit, dass die Nadel 3 unlösbar an dem Nadelträger 1 befestigt ist. Die Nadel 3 ist aus einem entsprechend für Injektionsnadeln geeigneten Metall und der Nadelträger 1 bevorzugt aus Kunststoff.

Der Nadelträger 1 weist einen Abschnitt 8 des Fluidkanals auf, der sich an das proximale Ende der Injektionsnadel 3 anschließt. Der Abschnitt 8 kann bevorzugt hohlzylindrisch sein. Insbesondere weist der Abschnitt 8 in etwa den gleichen Fluidführungsquerschnitt, insbesondere den gleichen Innendurchmesser wie die Injektionsnadel 3 auf. Durch diese Gestaltung kann beim Übergang des Fluids von Abschnitt 8 in den Abschnitt 10 der Injektionsnadel 3 die Gefahr einer Wirbelbildung oder turbulenten Strömung verringert werden.

Der Nadelträger 1 weist ferner einen Abschnitt 7 des Fluidführungskanals auf, der einen größeren Fluidführungsquerschnitt aufweist als die Abschnitte 8 und 10 des Fluidführungskanals. Der Abschnitt 7 verjüngt sich in Richtung Injektionsnadel 3, d.h. dass sich der Fluidführungsquerschnitt in Abschnitt 7 in Richtung Nadel 3 verringert. Die Verringerung kann z.B. mit einer konstanten Rate, d.h. einem konstanten Gradienten oder wie hier dargestellt mit einem zunehmenden und/oder abnehmende Gradienten erfolgen. In Richtung Injektionsnadel 3 erfolgt zunächst eine Verringerung des Fluidführungsquerschnitts mit einem zunehmenden Gradienten. Anschließend erfolgt eine Verjüngung des Fluidführungsquerschnitts mit einem abnehmenden Gradienten. Im Längsschnitt kann der Abschnitt 7 des Fluidkanals eine konkave und/oder eine konvexe Wölbung der Kanalwand aufweisen. In Richtung zur Injektionsnadel 3 weist die Wandung des Abschnitts zunächst eine konkave Wölbung und anschließend eine konvexe Wölbung auf. Die Übergänge zwischen den einzelnen Abschnitten, wie z.B. der Übergang von konkav auf konvex, kann stetig oder zumindest ohne scharfe Kante ausgestaltet sein. Gleiches gilt für den Übergang des Abschnitts 7 in den Abschnitt 8.

Der Nadelträger weist einen sich in distale Richtung erstreckenden, z.B. zylindrischen Schaft auf, in dem die Nadel 3 angeordnet und befestigt ist.

Der Nadelträger 1 ist mit einer Fügeverbindung 9 mit dem Fortsatz 2 verbunden. Die Fügeverbindung kann kraft-, stoff- oder formschlüssig sein. Insbesondere ist die Fügeverbindung fluiddicht. Eine besonders geeignete Fügeverbindung kann durch Verkleben, Verpressen, Verrasten oder Verschweißen erzielt werden.

Der mit dem Nadelträger 1 verbundene Fortsatz 2 weist einen Abschnitt 6 des Fluidführungskanals 6, 7, 8, 10 auf. Der Abschnitt 6 weitet sich in Richtung Injektionsnadel 3 auf, wie z.B. trichter- oder kegelförmig. Ein Mass für die Aufweitung kann ein Kegelwinkel von ca. 4° sein, um nur ein Beispiel zu nennen. Der Kegelwinkel kann z.B. aus einem Bereich von 1-45° sein, wobei eher geringere Kegelwinkel bevorzugt werden.

Bevorzugt ist, dass der Abschnitt 6 in den Abschnitt 7 mündet, wenn der Nadelträger 1 mit dem Fortsatz 2 verbunden ist. Die Abschnitte 6 und 7 können an der Mündung jeweils einen gleichen Fluidführungsquerschnitt, insbesondere Innendurchmesser, aufweisen.

Der Abschnitt 6 kann z.B. ein Querschnittsverhältnis von seinem größten Fluidführungsquerschnitt zu seinem kleinsten Fluidführungsquerschnitt von 4:1 aufweisen. Als vorteilhaft haben sich Querschnittsverhältnisse von 2:1 bis 10:1 herausgestellt. Das Verhältnis kann auch 2,5:1 betragen.

Das zu verabreichende Fluid wird von der Umgebung des Fortsatzes 2 über eine Öffnung 4 dem Fluidkanal 6, 7, 8, 10 zugeführt. Obwohl grundsätzlich eine Öffnung 4 ausreichen würde, sind in dem Beispiel vorteilhafterweise zwei Öffnungen 4 vorgesehen, die einander gegenüberliegend und seitlich des Fluidkanals 6 angeordnet sind. Ein Teil des Fluidkanals 6 ist somit zwischen den Öffnungen 4 angeordnet. Die Öffnungen 4 haben die Gestalt eines Langlochs, das z.B. zwischen 1 und 4 mm lang sein kann, wie z.B. 3 mm, und eine Breite wie der Durchmesser des zwischen den Öffnungen angeordneten Teils des Fluidkanals 6 aufweisen kann. Bevorzugt kann die Gesamtquerschnittsfläche der Öffnungen 4 größer sein als der kleinste Fluidführungsquerschnitt des Abschnitts 6. Vorteilhaft ist bereits der Querschnitt einer Öffnung 4 größer. Das Verhältnis der Gesamtquerschnittsfläche der Öffnungen 4 und des kleinsten Fluidführungsquerschnitts des Abschnitts 6 kann z.B. 7:1 betragen. Zumindest sollte die Gesamtquerschnittsfläche der Öffnungen 4 genauso groß wie der kleinste Fluidführungsquerschnitt sein. Das Querschnittsverhältnis kann sich z.B. in einem Bereich von 2:1 bis 10:1 bewegen.

Der Fortsatz 2 weist eine rotationssymmetrische Spitze auf, die in diesem Beispiel als Kegel ausgestaltet ist und das distale Ende des Fortsatzes 2 bildet. Die Anordnung der beiden Öffnungen 4 bewirkt, dass das Fluid seitlich, d.h. quer zur Längsachse, zugeführt und anschließend im Fluidführungskanal 6, 7, 8, 10 entlang der Längsachse in Richtung Nadel 3 transportiert wird.

Der Fortsatz 2 weist einen Verbindungsabschnitt 12 auf, mit dem die Einheit aus Nadelträger 1, Fortsatz 2 und Nadel 3 gegebenenfalls mittelbar oder unmittelbar mit einem Teil der Injektionsvorrichtung verbindbar ist. Alternativ könnte der Nadelträger 1 den Abschnitt 12 aufweisen.

Das proximale Ende des Nadelträgers 1 weist im Bereich des Fluidkanals 7 eine Vertiefung auf, die von einem ringförmigen Vorsprung umgeben wird. Der ringförmige Vorsprung ist in diesem Beispiel konzentrisch mit der Längsachse der Nadeleinheit 100. Der Fortsatz 2 weist an seinem distalen Ende eine Form auf, die in etwa einer Negativform der proximalen Stirnseite des Nadelträgers 1 entspricht. Insbesondere weist die distale Stirnseite des Fortsatzes 2 eine Ringnut, in die beim Zusammenfügen des Fortsatzes 2 mit dem Nadelträger 1 der ringförmige Vorsprung angeordnet wird, und einen kegelstumpfförmigen Vorsprung, der beim Zusammenfügen in die Vertiefung des Nadelträgers 1 eingreift, auf. Hierdurch wird bewirkt, dass eine besonders vorteilhafte Fügeverbindung 9, die insbesondere auch fluiddicht ist, erzielt wird. Durch das Ineinandergreifen der beiden Stirnseiten wird die für eine Fügeverbindung 9 nutzbare Oberfläche vergrößert.

Bei der Injektionsnadel 3 kann es sich um Nadeln mit einer großen Bandbreite von Durchmessern, Wandstärken und Längen handeln. Z.B. kann eine 23 Gauge Nadel genauso vorgesehen werden wie eine 31 Gauge Nadel. Die injizierbare Länge der Injektionsnadel 3 kann z.B. für eine subkutane oder transkutane Injektion vorgesehen sein.

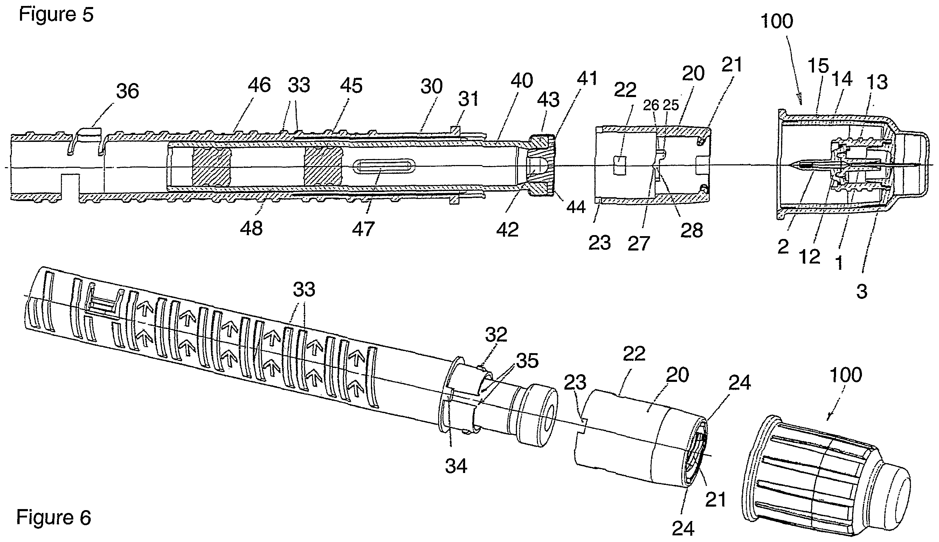

Insbesondere wird in den Figuren 5 und 6 eine vollständige Nadeleinheit 100 gezeigt. Die Einheit aus Nadelträger 1, Fortsatz 2 und Injektionsnadel 3 ist über den Verbindungsabschnitt 12 mit einem Befestigungsabschnitt 13 verbunden. Der hülsenförmige Befestigungsabschnitt 13 dient dazu, mit einem Befestigungsglied 20 verbunden zu werden und weist hierzu ein Gewinde, insbesondere ein Außengewinde auf. Die den Fluidkanal 6, 7, 8, 10 bildende Einheit ist axial fest mit dem Befestigungsabschnitt 13 verbunden. Je nach Anwendung kann die Einheit 1, 2, 3 relativ zu dem Befestigungsabschnitt 13 drehbar oder drehfest sein. Die Einheit 1, 2, 3 weist einen Verbindungsvorsprung 12a auf, der in eine am inneren Umfang des Befestigungsabschnitts 13 gebildete Ringnut 13c eingreift. Der Befestigungsabschnitt 13 ist bevorzugt dreh- und axial fest mit einem Gehäuse 14 verbunden. Grundsätzlich könnten der Befestigungsabschnitt 13 und das Gehäuse 14 auch einteilig gebildet sein, wobei die Mehrteiligkeit Vorteile bei der Herstellung der Nadeleinheit 100 hat. Das hülsenförmige Gehäuse 14 umgibt umfangsseitig sowohl den Befestigungsabschnitt 13 als auch den Fortsatz 2 und dessen Spitze. Das Gehäuse 14 erstreckt sich in proximale Richtung zumindest bis zur Spitze oder wie hier gezeigt bis über die Spitze hinaus, so dass die Spitze des Fortsatzes 2 nicht über das proximale Ende des Gehäuses 14 hervorsteht. Durch den Überstand des Gehäuses 14 über die Spitze des Fortsatzes 2 wird die Gefahr eines versehentlichen Stechens durch die Spitze 2 verringert. Zwischen dem Befestigungsabschnitt 13 und dem Gehäuse 14 wird ein Ringspalt gebildet. Das Gehäuse 14, der Befestigungsabschnitt 13 und die Einheit 1, 2, 3 sind konzentrisch zueinander angeordnet.

Das Gehäuse 14 weist an seinem distalen Ende eine Stirnfläche mit einer Öffnung auf, durch die die Injektionsnadel 3 mit einer bestimmten Länge hervortritt. Diese Länge entspricht im Wesentlichen der Injektionstiefe der Injektionsnadel 3, da das distale Ende des Gehäuses eine Anschlagfläche für den Körper des Patienten bildet. Das Gehäuse 14 kann z.B. aus Platzgründen eine Aussparung für den Nadelträger 1 aufweisen.

Die Nadeleinheit 100 weist ferner eine Verpackungshülse 15 auf, die in der in den Figuren 5 und 6 gezeigten Positionen über den äußeren Umfang des Gehäuses 14 konzentrisch angeordnet ist. Bevorzugt wird die Verpackungshülse 15 von dem Gehäuse 14 insbesondere reibschlüssig gehalten. Die Verpackungshülse 15 umgibt das Gehäuse 14 bevorzugt vollständig über den Umfang und auch über dessen Länge. Bevorzugt steht die Verpackungshülse 15 proximal über das proximale Ende des Gehäuses 14 zumindest ein Stück weit über. Die am proximalen Ende der Verpackungshülse 15 gebildete Öffnung, durch die das Gehäuse 14 mit der Einheit 1, 2, 3 einführbar oder herausziehbar ist, kann im Auslieferungszustand der Nadeleinheit 100 mit einer sog. Peelfolie verschlossen sein, um den Inhalt der Verpackungshülse 15 steril zu halten oder zumindest vor Verschmutzung zu schützen. Die Verpackungshülse 15 steht distal über das distale Ende der Nadel 3 über und ist dort stirnseitig verschlossen. Die Verpackungshülse 15 bildet insoweit einen geschlossenen Topf, der an seinem proximalen Ende eine Öffnung aufweist. Die Verpackungshülse 15 weist an ihrem äußeren Umfang eine Struktur auf, welche es einem Verwender der Vorrichtung besser ermöglicht, ein Drehmoment um die Längsachse auf die Verpackungshülse 15 aufzubringen. Da die Verpackungshülse 15 in ihrem auf dem Gehäuse 14 vollständig aufgesetzten Zustand zumindest reibschlüssig bevorzugt alternativ oder zusätzlich formschlüssig drehfest mit dem Gehäuse 14 verbunden ist, dreht sich das Gehäuse 14 zusammen mit dem Befestigungsabschnitt 13 mit der Verpackungshülse 15 mit, wenn ein Drehmoment auf die Verpackungshülse 15 aufgebracht wird.

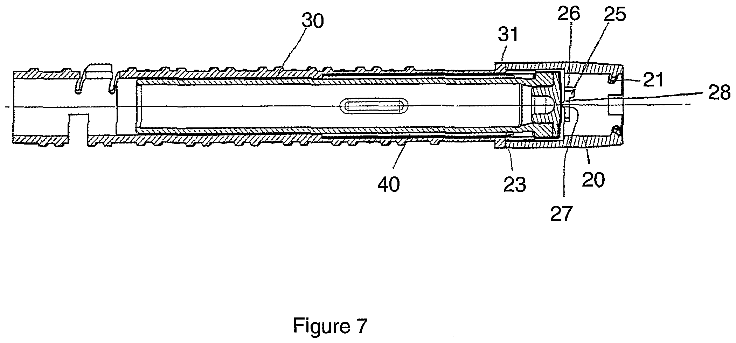

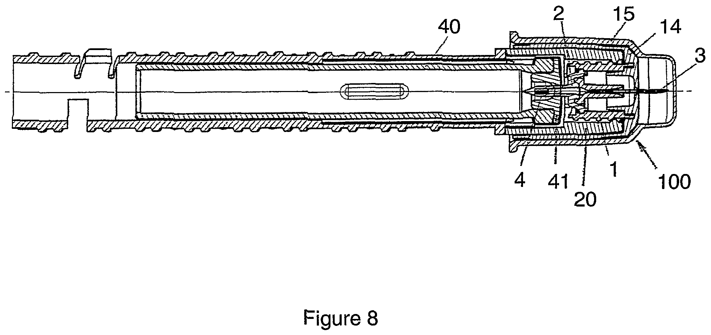

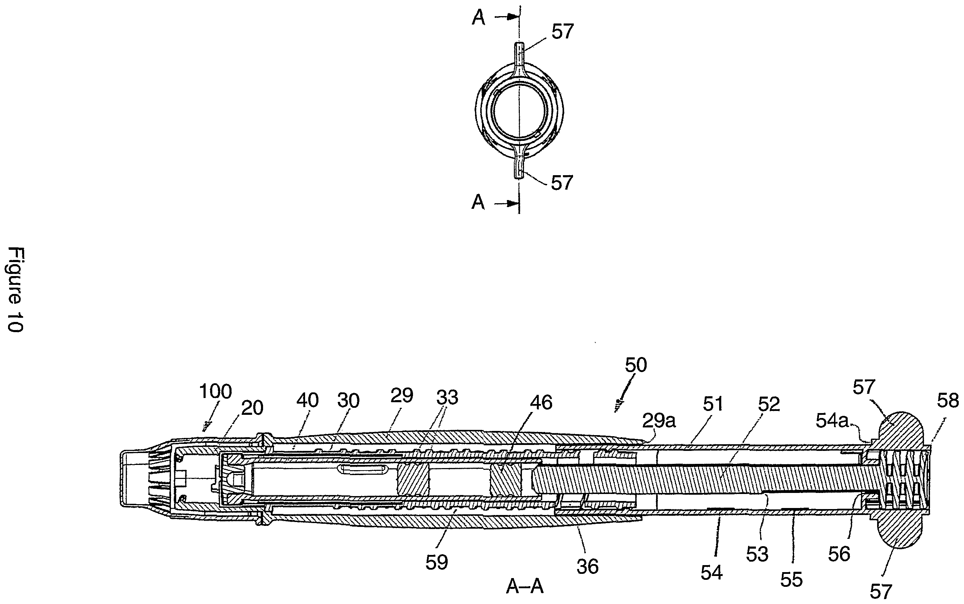

Die Nadeleinheit 100 kann an eine Injektionsvorrichtung 50 angebracht werden (Figur 10). Teile der Injektionsvorrichtung, nämlich eine Ampullenhalterung 30 und ein Befestigungsglied 20, werden zusammen mit einer Ampulle 40 in den Figuren 5 und 6 vereinzelt, in Figur 7 zusammengesetzt und in Figur 8 zusammengesetzt mit einer aufgesetzten Nadeleinheit 100 gezeigt.

Bei der Ampulle 40, wie sie z.B. in den Figuren 5 und 6 gezeigt wird, handelt es sich um eine sog. Zweikammerampulle, die auch als Zweikammerkarpule bezeichnet werden kann, deren Besonderheit es ist, dass ein zu verabreichendes Produkt unmittelbar vor der Verabreichung aus zwei Komponenten zusammengemischt wird, die zur Lagerung in zwei verschiedenen Kammern enthalten sind. Z.B. kann die distale Kammer, d.h. die Kammer, die zwischen einem Verschluss 41, 42, 43, 44 und einem ersten Kolben 45 angeordnet ist, mit einem Feststoff wie z.B. einem körnigen oder pulverförmigen Material teilweise oder ganz gefüllt sein. Proximal des ersten Kolbens 45 befindet sich eine zweite Kammer, die zwischen dem ersten Kolben 45 und einem zweiten Kolben 46 angeordnet ist. In dieser Kammer kann sich z.B. ein flüssiger Bestandteil des Medikaments befinden. Beide Kolben 45, 46 liegen dichtend an einem hohlzylindrischen Gehäuseabschnitt 48 der Ampulle 40 an und können entlang dem Gehäuseabschnitt 48 verschoben werden.

Die Ampulle 40 weist im Bereich des Gehäuseabschnitts einen sog. Bypass 47 auf, der nach aussen eine Auswölbung bildet. Der Bypass 47 ist axial länger als der erste Kolben 45.

Zum Abmischen wird distal gerichtet ein Druck auf den zweiten Kolben 46 ausgeübt, der über das flüssige Produkt in der Kammer zwischen dem ersten Kolben 45 und dem Kolben 46 auf den Kolben 45 übertragen wird. Wenn der zweite Kolben 46 in distale Richtung verschoben wird, wird somit auch der ersten Kolben 45 verschoben und zwar bis in den Bereich des Bypasses 47. Durch die Auswölbung des Bypasses 47 kann, sofern sich der Kolben 45 vollständig im Bereich des Bypasses 47 befindet, das flüssige Produkt an dem Kolben 45 vorbei fließen in die distale Kammer und sich mit dem darin befindlichen Produktbestandteil mischen. Das Mischen kann vom Verwender durch Schütteln unterstützt werden. Das flüssige Produkt ist vollständig aus der proximalen Kammer beseitigt, wenn der zweite Kolben 46 an den ersten Kolben 45 anschlägt. Das Gemisch aus den beiden Produktbestandteilen kann eine homogene oder heterogene Mischung bilden. Die heterogene Mischung eines flüssigen Bestandteils mit Festkörperpartikeln wird allgemein als Dispersion, im Speziellen als Suspension bezeichnet. Das zu verabreichende Produkt kann dann über den Fluidkanal 6, 7, 8, 10 der Nadeleinheit 100, deren Öffnungen 4 sich innerhalb der Ampulle wie z.B. im Bereich 42 befinden, ausgeschüttet werden, indem die Kolben 45 und 46 in distale Richtung entlang des hohlzylindrischen Gehäuseabschnitts 48 verschoben werden. Die Ampulle 40 weist einen Verschluss 41, 42, 43, 44 auf, der ein von dem Fortsatz 2 durchstechbares Septum 41 aufweist. Solche Septen können aus Gummi oder Kautschuk oder einem anderen geeigneten Kunststoff sein.

Das Septum 41 weist einen scheibenförmigen Abschnitt und einen sich daran in proximale Richtung anschließenden hohlzylindrischen Abschnitt auf. Der hohlzylindrische Abschnitt bildet in seinem Inneren eine Ausnehmung, in der in bevorzugten Ausführungen die Öffnungen 4 der vollständig aufgesetzten Nadeleinheit 100 platziert sind. Die Außenseite des hohlzylindrischen Abschnitts des Septums 41 zentriert das Septum 41 am inneren Umfang der Ampulle 40, insbesondere am distalen Ende der Ampulle 40. Am distalen Ende der Ampulle 40 ist ein ringförmig umlaufender Wulst gebildet, der radial nach außen ragt und allgemein als Abragung bezeichnet werden kann. Der Wulst 43 steht radial über den äußeren Umfang des hohlzylindrischen Abschnitts 48 der Ampulle 40. Mit anderen Worten weist die Ampulle 40 an der Stelle des Wulsts 43 einen größeren Außendurchmesser auf als im Bereich des hohlzylindrischen Abschnitts 48. Der Außendurchmesser des Wulsts 43 bildet in diesem Beispiel auch den größten Außendurchmesser der Ampulle 40. Der Wulst 43 ist einteilig mit dem hohlzylindrischen Abschnitt 48 verbunden. Dieser Teil der Ampulle kann aus Kunststoff oder aus Glas gebildet sein. Der scheibenförmige Teil des Septums 41 weist in etwa den gleichen Außendurchmesser auf wie der Wulst 43. Septum 41 und Wulst 43 werden zusammengehalten durch ein Umformteil 44, das z.B. aus einem Metallblech gefertigt sein kann. Dieses Teil kann auch als Krone bezeichnet werden. Das Umformteil 44 umgreift das Septum 41 und den Wulst 43 über den Umfang und sowohl distal des Septums 41 als auch proximal des Wulsts 43. Durch das Umformteil 44 werden Wulst 43 und Septum 41 axial fest verbunden, d.h. fluiddicht zusammengehalten. An der distalen Stirnseite weist das Umformteil 44 eine Öffnung auf, die z.B. kreisförmig sein kann und durch die der Fortsatz 2 durch das Septum 41 in das Innere der bevorzugt bereits abgemischten Ampulle 40 eingeführt werden kann.

Die Ampulle 40 weist zwischen dem hohlzylindrischen Teil 48 und dem Wulst 43 einen gegenüber dem Außendurchmesser des Abschnitts 48 eingeschnürten Hals auf, so dass das Umformteil 44 noch besser, insbesondere weiter nach innen, um das proximale Ende des Wulsts 43 umformbar ist. Das proximale Ende der Ampulle 40 ist offen, so dass eine Kolbenstange 52 (Figur 10) in die Ampulle 40 und auf den ersten Kolben 46 schiebbar ist.

An der Ampullenaufnahme 30 kann das Befestigungsglied 20 befestigt sein oder werden. Befestigungsglied 20 und Ampullenaufnahme 30 werden vorzugsweise als Ampullenhalterung bezeichnet. Das Befestigungsglied 20 ist hülsenförmig und dreh- und axialfest mit der Ampullenaufnahme 30 verbunden, insbesondere verrastet. Die Drehfestigkeit wird durch den Eingriff der Aussparung 23 in den Nocken 34 erzielt. Die axial feste Anordnung des Befestigungsglieds 20 an der Ampullenaufnahme 30 wird durch das Einrasten der Nocken 32 in die Fenster 22 erzielt.

Wie aus den Figuren 5 und 6 erkennbar ist, ist die Ampulle 40 über eine distale Öffnung der hülsenförmigen Ampullenaufnahme 30 in diese eingeführt. Der Außendurchmesser des Verschlusses 41, 42, 43, 44 ist größer als der Innendurchmesser der Ampullenaufnahme 30, so dass der Verschluss am distalen Ende der Ampullenaufnahme 30 anliegt. Wie insbesondere aus Figur 7 erkennbar ist, ist der Verschluss der Ampulle 40 zwischen der Ampullenaufnahme 30 und dem Befestigungsglied 20 axial eingefasst und somit axial fixiert. Die Einfassung kann beispielsweise ein geringes axiales Spiel der Ampulle 40 zulassen. Bevorzugt ist es jedoch, dass die Einfassung dergestalt ist, dass das axiale Spiel weiter verringert wird. Hierzu weist das distale Ende der Ampullenaufnahme 30 wenigstens eine Abragung, in diesem Fall vier Abragungen 35 auf, die sich in distale Richtung erstrecken und an denen der Verschluss 41, 42, 43, 44 anliegt. Die mindestens eine Abragung 35 kann starr, wie hier gezeigt, oder federnd an der Ampullenhalterung, insbesondere an der Ampullenaufnahme, angeordnet sein. Die federnde Anordnung hat zudem den Vorteil, dass ein axiales Spiel so gut wie nicht mehr vorhanden ist. Die federnde Anordnung kann dadurch erzielt werden, dass proximal der mindestens einen Abragung 35 ein sich in Umfangsrichtung erstreckender Schlitz vorgesehen ist, so dass der sich zwischen Schlitz und Abragung 35 befindliche Steg in Axialrichtung federn kann.

Der Verschluss 41, 42, 43, 44 kann von der distalen Seite her durch eine am inneren Umfang des Befestigungsglieds 20 gebildete Schulter eingefasst werden. Die Schulter kann teilweise oder vollständig über den Umfang umlaufen. Bevorzugt ist eine sich von der inneren Umfangsfläche nach innen erstreckende Abragung 28 vorgesehen, entweder alternativ oder zusätzlich zu der Schulter und kann als Einfassung für den Verschluss 41, 42, 43, 44 dienen. Die Abragung 28 weist an ihrer zum Verschluss 41, 42, 43, 44 hinweisenden Seite eine Abragung, insbesondere einen Nocken 27 auf, der gegen die distale Stirnfläche des Verschlusses drückt, insbesondere gegen das Umformteil 44. Durch die punktuelle Belastung des Nockens 27 kann sich ein Teil des Verschlusses, wie z.B. das Umformteil 44 elastisch oder plastisch verformen, wodurch eine sichere, spielarme Einfassung des Verschlusses 41, 42, 43, 44 zwischen der Ampullenaufnahme 30 und dem Befestigungsglied 20 erreicht wird. Der Nocken 27 kann starr oder in Längsrichtung federnd angeordnet sein.

Wie aus den Figuren 5-7 erkennbar ist, weist die Ampullenaufnahme 30 an ihrem äußeren Umfang ein Außengewinde 33 auf, mit dem die Ampullenhalterung in ein Gehäuse 51 der Injektionsvorrichtung 50 (Figur 10) einschraubbar ist, wobei das Gehäuse 51 ein entsprechendes Innengewinde für das Außengewinde 33 der Ampullenhalterung aufweist. Um dem Verwender die intuitive Handhabung der Vorrichtung 50 zu erleichtern, kann an der Ampullenhalterung 20, 30, insbesondere am Umfang der Ampullenaufnahme 30 mindestens ein Pfeil angeordnet sein, der in die Drehrichtung zum Abmischen der Vorrichtung weist. Bevorzugt sind eine Vielzahl von Pfeilen über die Länge der Ampullenaufnahme 30 verteilt. Die Pfeile können z.B. aufgedruckt oder vorzugsweise als Erhebungen oder Vertiefungen, die insbesondere bereits bei der Spritzgussformung der Ampullenaufnahme 30 geformt werden, ausgebildet sein. Der mindestens eine Pfeil kann zwischen benachbarten Gewindegängen 33 angeordnet sein.

Die Ampullenhalterung, insbesondere die Ampullenaufnahme 30 weist ferner ein in radiale Richtung federnd gelagertes Rastglied 36 auf. Die Federung wird mittels eines sich in Umfangsrichtung erstreckenden Arms, der an der Ampullenhalterung 30 gebildet ist und an dem das Rastglied 36 gebildet ist, bewirkt. Die Funktion dieses Rastglieds 36, insbesondere Nockens, wird später mit Bezug auf Figur 10 beschrieben.

Das Befestigungsglied 20 weist ferner ein ein- oder mehrgängiges Gewinde, in diesem Beispiel in der Gestalt von zwei Gewindeabschnitten 21 auf, die es ermöglichen, dass die Nadeleinheit 100 mit dem auf dem Befestigungsabschnitt 13 befindlichen Außengewinde auf die Injektionsvorrichtung aufgeschraubt werden kann. Durch die Axialbewegung des Fortsatzes 2 beim Aufschrauben durchsticht die Spitze des Fortsatzes 2 das Septum 41. Das für das Aufschrauben erforderliche Drehmoment wird über die Verpackungshülse 15 oder das Gehäuse 14 aufgebracht. Nachdem die Nadeleinheit 100 vollständig aufgeschraubt ist, nimmt sie die in Figur 8 gezeigte Position ein. Die Öffnungen hier befinden sich nun innerhalb der Ampulle 40, so dass das distale Ende der Injektionsnadel 3 fluidisch mit dem Innern der Ampulle 40 verbunden ist. Ein distaler Abschnitt des Befestigungsglieds 20 befindet sich nun im Ringspalt zwischen dem Befestigungsabschnitt 13 und dem Gehäuse 14.

Sofern die Einheit 1, 2, 3 relativ zum Befestigungsabschnitt 13 drehbar angeordnet ist, besteht die Möglichkeit, dass der Fortsatz mit einer reinen Axialbewegung in das Septum 41 einsticht. Dies kann Vorteile hinsichtlich der Abdichtung zwischen Septum und Fortsatz ergeben. Bei einer relativ zum Befestigungsabschnitt 13 drehfest angeordneten Einheit 1, 2, 3 wird das Septum vom Fortsatz 2 mittels einer kombinierten Dreh- und Axialbewegung durchstochen, was Vorteile bietet hinsichtlich des für das Aufschrauben benötigten Drehmoments.

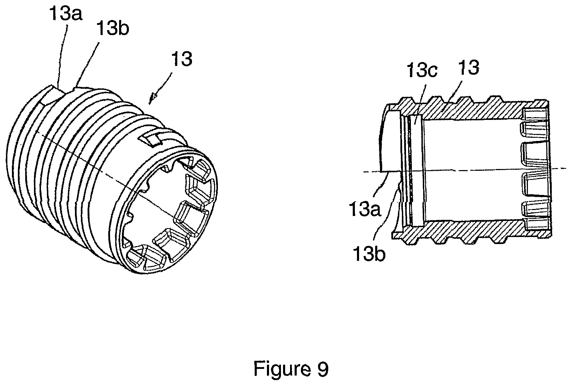

Wie aus den Figuren 5 und 9 erkennbar ist, weisen das Befestigungsglied 20 einen in Umfangsrichtung wirkenden Anschlag 26 und der Befestigungsabschnitt 13 einen ebenfalls in Umfangsrichtung wirkenden Anschlag 13a auf. Es können auch mehrere, wie in diesem Beispiel zwei solche Anschläge jeweils am Befestigungsglied 20 als auch am Befestigungsabschnitt 13 angeordnet sein.

Die Anschläge 26 und 13a bilden ein Anschlagpaar und sind so positioniert, dass sie in der vollständig aufgeschraubten Position der Nadeleinheit 100 (Figur 8) in einen Drehanschlag geraten, wodurch ein Weiterdrehen auf eine sichere Weise verhindert wird. Der Anschlag 13a ist am proximalen Ende des Befestigungsabschnitts 13 gebildet. Der Anschlag 26 ist an der Abragung 28 des Befestigungsglieds 20 gebildet. Optional könnte auch ein Axialanschlag statt eines Drehanschlags vorgesehen sein, wobei der Drehanschlag gegenüber einem Axialanschlag den Vorteil hat, dass die in Anschlag geratenen Teile weniger belastet werden. Denn durch die von der Gewindesteigung erzeugte Übersetzung ist mit einem bestimmten Drehmoment in Axialrichtung eine deutlich höhere Kraft erzielbar als in Umfangsrichtung. Insbesondere kann das Befestigungsglied 20 eine oder mehrere Abragungen 24 oder 25 aufweisen, die kurz vor dem Erreichen des Drehanschlags mit der Nadeleinheit 100 verrasten, so dass eine Rückdrehung der Nadeleinheit 100 in entgegen gesetzte Richtung nur mit einem erhöhten Drehmoment oder gar nicht, d.h. nur durch Zerstörung eines Bauteils oder eines Teils eines Bauteils möglich ist. Alternativ können statt Abragungen 24, 25 Ausnehmungen vorgesehen sein, die dem gleichen Zweck dienen. Durch das Verrasten beim Aufschrauben erhält der Verwender zum einen ein taktiles Signal, das ihm anzeigt, dass die Nadeleinheit 100 nun vollständig aufgeschraubt ist. Zum andern verhindert es eine unbeabsichtigte Rückdrehung, so dass die Gefahr einer Fehlanwendung verringert werden kann. Sofern als Rückdrehsicherung Ausnehmungen oder Nocken 24 vorgesehen sind, greifen diese in das Gehäuse 14 der Nadeleinheit 100 ein, insbesondere in dessen im Ringspalt gebildete Stirnseite. Die Stirnseite kann hierzu mindestens eine Abragung, wie z.B. Rippen oder einen Nocken oder eine Ausnehmung aufweisen, die kurz vor oder beim Erreichen des Drehanschlags überfahren wird.

Alternativ oder zusätzlich kann eine Abragung 25 in der Gestalt eines in distale Richtung ragenden Nockens vorgesehen sein, der kurz vor oder beim Erreichen des Drehanschlags eine Abragung 13b, insbesondere einen Nocken, überfährt. Die Abragung 13b ist an der proximalen Stirnseite des Befestigungsabschnitts 13 und in Umfangsrichtung versetzt vor dem Anschlag 13a angeordnet. Der Abstand zwischen der Abragung 13b und dem Anschlag 13a kann insbesondere in etwa der in Umfangsrichtung gemessenen Breite der Abragung 25 entsprechen, wodurch erreicht wird, dass der Nocken 25 in einem vollständig aufgeschraubten Zustand zwischen dem Anschlag 13a und der Abragung 13b gehalten ist.

Eine Abragung 13b, die eine Schraubbewegung entgegen der Aufschraubrichtung zulässt, kann in Umfangrichtung beidseits abgeflachte Flanken aufweisen. Eine Abragung 13b, die verhindern soll, dass eine Drehung entgegen der Aufschraubrichtung möglich ist, kann beispielsweise die Form eines Sägezahns aufweisen, der ein Überfahren der Abragung 13b erlaubt aber eine Rückdrehung durch die steile Fläche des Sägezahns verhindert. Gleiches gilt selbstverständlich für die Verrastung der Ausnehmungen oder Abragungen 24 mit den Ausnehmungen oder Abragungen an dem Gehäuse 14.

Die Abragung 13b ist auf der als schraubenförmige Bahn ausgestalteten Stirnseite des Befestigungsabschnitts angeordnet. Die Schraubenform weist in etwa die Steigung des Gewindes des Befestigungsabschnitts 13 auf.

Eine bevorzugte Ausführungsform einer Injektionsvorrichtung bei der die beschriebene Erfindung Anwendung finden kann, ist in Figur 10 gezeigt. Bei der Vorrichtung handelt es sich um eine Abmisch- und Ausschüttvorrichtung 50, die im Folgenden der Einfachheit halber nur als Injektionsvorrichtung bezeichnet wird. Die Injektionsvorrichtung 50 weist ein Gehäuse 51 auf mit einem Innengewinde, in welches die in Figur 7 gezeigte Ampullenhalterung 20, 30, welche ein Außengewinde 33 aufweist, einschraubbar ist. Innerhalb des hülsenförmigen Gehäuses 51 ist eine Kolbenstange 52 angeordnet, die mit einem Injektionsknopf 58 verbunden ist. Der Injektionsknopf 58 ist in einem Ausgangszustand der Injektionsvorrichtung 50, d.h. vor einer Abmischung der Produktbestandteile der Zweikammerampulle 40 auf seiner axialen Länge insbesondere vollständig von dem Gehäuse 51 umgeben. An dem Gehäuse 51 sind zwei radial nach aussen weisende Flügel 57 angeordnet. Durch die plättchenförmigen Flügel wird dem Verwender der Vorrichtung das Halten und das Aufbringen eines notwendigen Drehmoments beim Abmischen erleichtert. Die Flügel 57 dienen somit als Greifhilfe und verbessern vorzugsweise im Zusammenspiel mit einem Greifabschnitt 29 die intuitiv richtige Handhabung. Die Flügel 57 befinden sich am proximalen Ende der Vorrichtung 50.

Die Injektionsvorrichtung kann in einer bevorzugten Ausführungsform eine äußere Hülse 29, die als Greifabschnitt dient, aufweisen, die der Verwender der Vorrichtung 50 mit einer Hand umgreifen kann, während er mit der anderen Hand an den Flügeln 57 das Gehäuse 51 auf die Ampullenhalterung, insbesondere in den zwischen der äußeren Hülse 29 und der Ampullenhalterung 30 gebildeten Ringspalt schraubt. Hierdurch wird der Vorteil erzielt, dass Patienten, die über eingeschränkte motorische Fähigkeiten verfügen, ein sicheres Abmischen der Produktbestandteile erlaubt wird. Am Ende der Abmischsequenz und gegebenenfalls einer Primesequenz geraten der am proximalen Ende der äußeren Hülse 29 gebildete Anschlag 29a und der vom Gehäuse 51 im Bereich des distalen Endes gebildete Anschlag 54a in einen Dreh- oder Axialanschlag, so dass dem Benutzer z.B. hierdurch angezeigt wird, dass der Abmischvorgang und Primevorgang beendet sind.

In einer alternativen, ebenfalls bevorzugten Ausführung kommt die Injektionsvorrichtung 50 ohne die äußere Hülse 29 aus. Der Verwender der Injektionsvorrichtung kann zur Aufbringung des Drehmoments die Verpackungshülse 15 oder das Gehäuse 14 mit der einen Hand und mit der anderen Hand das Gehäuse 51 oder gegebenenfalls daran befestigte Flügel 57 umgreifen. Die Abmischung funktioniert analog zur Ausführung mit einem hülsenförmigen Greifabschnitt 29, wobei das Ende des Abmischvorgangs z.B. durch den Dreh- oder Axialanschlag des distalen Endes des Gehäuses 51 an den in Figur 5 mit dem Bezugszeichen 31 bezeichneten ringförmig umlaufenden Kragen der Ampullenhalterung 20, 30 angezeigt wird. Diese Variante ist auch bei der Ausführung mit einer äußeren Hülse 29 möglich. Aufgrund der Drehanschläge 26, 13a zwischen Nadeleinheit 100 und Ampullenhalterung 20, 30 kann eine Überlastung der am Anschlag beteiligten Bauteile sicher verhindert werden, auch wenn das Drehmoment zum Abmischen über die Nadeleinheit 100 läuft.

Für beide Ausführungsformen einer Injektionsvorrichtung 50 gilt, dass beim Ineinanderschrauben der Ampullenaufnahme 30 und des Gehäuses 51 beim Abmischen der Produktbestandteile die Kolbenstange 52 in einen Anschlag mit dem Kolben 46 gerät, wodurch sich das Gehäuse 51 relativ zu der Kolbenstange 52 in distale Richtung bewegt. Die Kolbenstange 52 bleibt hierbei axial fest relativ zur Ampulle 40 aufgrund der Haftreibung zwischen dem/den Kolben 45, 46 und der Ampullenwand. Die Kolbenstange 52 weist eine Aussparung auf, an deren distalen Ende 53 ein Anschlag 53 gebildet ist. Während des Einschraubens bewegt sich der von dem Gehäuse 51 gebildete Axialanschlag 56 in Richtung Anschlag 53. Der Injektionsknopf 58 tritt bei dieser Bewegung proximal aus dem Gehäuse 51 hervor. Sobald der Anschlag 56 an den Anschlag 53 anstößt, wird die Kolbenstange 52 mitgenommen, so dass die Kolbenstange 52 die Axialbewegung des Gehäuses 51 in distale Richtung relativ zu der Ampullenhalterung 30 und zur Ampulle 40 mitmacht. Die Kolbenstange 52 kann nun den Kolben 46 in Richtung distal verschieben, wodurch die Abmischsequenz durchgeführt wird, wie weiter oben beschrieben wurde. Am Ende des Abmischens kann das Rastglied 36 in ein Rastelement 54 eingreifen oder dieses überfahren, wodurch dem Verwender das Ende des Abmischvorgangs angezeigt werden kann. Der Verwender schüttelt nun die Vorrichtung 50, so dass sich die Produktbestandteile vermischen. Anschließend kann er die Vorrichtung primen, um eventuell in der Ampulle 40 noch enthaltene Luft aus der Ampulle 40 zu entfernen. Hierzu kann der Verwender die Schraubbewegung des Gehäuses 51 relativ zu der Ampullenhalterung 30 fortsetzen, wobei dann beide Kolben verschoben werden und das Volumen im Produktbehältnis verkleinert wird, wodurch die Luft aus dem Produktbehältnis 40 über den Fluidkanal 6, 7, 8, 10 der Nadeleinheit 100 ausgestoßen wird. Am Ende der Primesequenz kann das Rastglied 36 in ein weiteres ebenfalls am inneren Umfang des Gehäuses 51 gebildetes Rastelement 55 einrasten, wodurch dem Verwender das Ende der Primesequenz angezeigt wird. Die Vorrichtung 50 ist nun bereit für eine Produktausschüttung, die nach dem Einstechen der Nadel 3 in eine gewünschte Körperstelle durch Drücken des Injektionsknopfs 58 in distale Richtung relativ zum Gehäuse 51 und zur Ampulle 40 bewirkt wird.

Die Rastelemente 54 und 55 können so gebildet sein, dass bei einer Wechselwirkung, insbesondere beim Überfahren des Rastglieds 36 über die Rastelemente 54 und 55 ein akustisches oder taktiles Signal erzeugt wird. Ferner ist durch eine in Umfangrichtung asymmetrische Ausbildung der Flanken am Rastglied 36 realisierbar, dass nachdem das Rastglied 36 die Rastelemente 54 oder 55 erreicht hat, ein Weiterdrehen in eine der Abmisch- oder Primebewegung entgegengesetzte Richtung nicht mehr möglich ist.

Bei der Ausführungsform mit einer äußeren Hülse 29 kann diese dreh- und axial fest mit der Ampullenaufnahme 30 verbunden sein, wie z.B. durch eine Rastverbindung oder durch eine einteilige Ausbildung mit der Ampullenhalterung 30 oder dem Befestigungsglied 20. Die äußere Hülse 29 kann sich von dem Befestigungsglied 20 soweit in proximale Richtung erstrecken, dass ihr proximales Ende proximal über das proximale Ende der Ampulle 40 und insbesondere auch über das proximale Ende der Produktbehältnisaufnahme 30 ragt.

Die in Figur 10 gezeigte Ausführung hat den Vorteil, dass der Verwender der Vorrichtung 50 kein Gewinde sieht, was vor allem bei Menschen, die Technik skeptisch gegenüber stehen, von Vorteil ist. Z.B. kann auf der Außenseite des Gehäuses 51 eine Farbkodierung angegeben sein, aus der zusätzlich optisch ersichtlich ist, ob die Vorrichtung sich beim Abmischen oder beim Primen befindet oder ob die Vorrichtung fertig für eine Produktausschüttung ist. Z.B. kann eine erste Farbe so angeordnet sein, dass sie nach dem erfolgten Abmischen von der ersten Hülse 29 abgedeckt wird. Ferner kann eine zweite, andere Farbe vorgesehen sein, die nach dem Primen unter der zweiten Hülse 29 verschwindet. Beispielsweise kann nun eine dritte Farbe vorgesehen sein, die dem Verwender anzeigt, dass die Vorrichtung nun bereit ist für eine Produktausschüttung. Die äußere Hülse 29 kann z.B. mit einer reibungserhöhenden Struktur zur Verbesserung des Griffs versehen sein, wie z.B. Rippen oder Noppen oder ein reibungserhöhendes Material, wie z.B. eine Gummischicht. Gleiches gilt auch für die Flügel 57.

## Patentansprüche

1. Vorrichtung zum Abmischen von einem flüssigen Produkt und einem Feststoff zu einem injizierenden Produkt und zur Verabreichung des Produkts, umfassend
- ein Gehäuse (51) mit einem Greifabschnitt (57) zum Greifen mit einer Hand,
- eine Aufnahme (30), wobei das Gehäuse (51) in einem Gewindeeingriff mit der Aufnahme (30) ist,
- eine Zweikammerampulle (40), die von der Aufnahme (30) aufgenommen ist, die insbesondere von der distalen Stirnseite her in die Aufnahme (30) eingeführt ist,
wobei die Zweikammerampulle (40) einen Bypass (47), einen ersten (45) und einen zweiten Kolben (46) aufweist, wobei die beiden Kolben (45, 46) dichtend an einem hohlzylindrischen Gehäuseabschnitt (48) der Zweikammerampulle (40) anliegen und entlang des Gehäuseabschnitts (48) verschoben werden können, wobei die Zweikammerampulle (40) den Bypass (47) im Bereich des Gehäuseabschnitts (48) aufweist und eine nach aussen Auswölbung bildet, und der Bypass (47) axial länger als der erste Kolben (45) ist,
und
- eine äussere Hülse (29) mit einem Greifabschnitt zum Greifen mit einer anderen Hand oder eine äussere Hülse (29), welche als Greifabschnitt zum Greifen mit einer anderen Hand ausgebildet ist, wobei sich die äussere Hülse (29) über einen Teil der Länge der Aufnahme (30) und/oder der Zweikammerampulle (40) erstreckt und das Gehäuse (51) zwischen der äusseren Hülse (29) und der Aufnahme (30) angeordnet und relativ zu der Aufnahme (30) in die äussere Hülse (29) bewegbar ist, wobei die Bewegung eine kombinierte Axialbewegung und Drehbewegung ist und durch diese Bewegung das flüssige Produkt und der Feststoff in der Zweikammerampulle (40) zu dem injizierenden Produkt vermischt werden, wobei zum Abmischen ein Druck auf den zweiten Kolben (46) ausgeübt wird, der über das flüssige Produkt zwischen dem ersten Kolben (45) und dem zweiten Kolben (46) auf den ersten Kolben (45) übertragen wird, wobei der zweite Kolben (46) in distale Richtung bis der erste Kolben (45) in den Bereich des Bypasses (47) verschoben wird, wobei wenn sich der erste Kolben (45) vollständig im Bereich des Bypasses (47) befindet, das flüssige Produkt durch die Auswölbung des Bypasses (47) an dem ersten Kolben (45) vorbei fliesst und sich mit einem distal von dem ersten Kolben (45) angeordneten Feststoff zu dem injizierenden Produkt mischt.

2. Vorrichtung nach einem der vorhergehenden Ansprüche, **dadurch gekennzeichnet, dass** die Aufnahme (30) und die äussere Hülse (29) dreh- und axialfest verbunden sind.

3. Vorrichtung nach einem der vorhergehenden Ansprüche, **dadurch gekennzeichnet, dass** zwischen äusserer Hülse (29) und Aufnahme (30) ein Ringspalt gebildet ist, in den das zumindest ein Teil des Gehäuses (51) bewegbar ist.

4. Vorrichtung nach einem der vorhergehenden Ansprüche, **dadurch gekennzeichnet, dass** von dem Gehäuse (51) eine Kolbenstange (52) aufgenommen ist, die zumindest bei einem Teil der Bewegung des Gehäuses (51) in die äussere Hülse (29), insbesondere bei einem Abmischvorgang der Zweikammerampulle (40), relativ zum Gehäuse (51) axialfest ist.

5. Vorrichtung nach einem der vorhergehenden Ansprüche, **dadurch gekennzeichnet, dass** von dem Gehäuse (51) eine Kolbenstange (52) aufgenommen ist, die zumindest bei einem Teil der Bewegung des Gehäuses (51) in die äussere Hülse (29) relativ zum Gehäuse (51) axialbewegbar ist, wobei insbesondere die Kolbenstange (52) proximal aus dem Gehäuse (52) hervortritt.

6. Vorrichtung nach einem der vorhergehenden Ansprüche, **dadurch gekennzeichnet, dass** die Kolbenstange (52) in einem abgemischten Zustand der Zweikammerampulle (40) proximal aus dem Gehäuse (51) ragt, wobei die Kolbenstange (52) relativ zu dem Gehäuse (51) in distale Richtung verschiebbar ist.

7. Vorrichtung nach einem der vorhergehenden Ansprüche, **dadurch gekennzeichnet, dass** die Kolbenstange (52) einen sich über einen Teil ihrer Länge erstreckenden Eingriffsabschnitt aufweist, in den das Gehäuse (51) oder ein gehäusefestes Element eingreift und insbesondere nur eine begrenzte axiale Verschiebbarkeit der Kolbenstange (52) relativ zu dem Gehäuse (51) zulässt.

8. Vorrichtung nach einem der vorhergehenden Ansprüche, **dadurch gekennzeichnet, dass** das Gehäuse (51) mindestens eine an seinem äusseren Umfang angeordnete Abragung (57) aufweist, die insbesondere flügelförmig ist und sich mit ihrer schmalen Seite in Axialrichtung erstreckt.

9. Vorrichtung nach einem der vorhergehenden Ansprüche, **dadurch gekennzeichnet, dass** das Gehäuse (51) eine zylinderförmige, glatte Oberfläche aufweist.

10. Vorrichtung nach einem der vorhergehenden Ansprüche, **dadurch gekennzeichnet, dass** das Gehäuse (51) mindestens einen Informationsabschnitt aufweist, der bei der Bewegung des Gehäuses (51) in die äussere Hülse (29) nicht erblickbar ist, z.B. unter der äusseren Hülse (29) verschwindet, oder z.B. durch ein Fenster der äusseren Hülse (29) erblickbar ist.

11. Vorrichtung nach einem der vorhergehenden Ansprüche, **dadurch gekennzeichnet, dass** die Aufnahme (30) ein Rastelement (36) aufweist, das in mindestens einer Position des Gehäuses (51) in Bezug auf die Aufnahme (30) mit mindestens einem Rastgegenglied (54, 55) wechselwirkt.

## Claims

1. A device for mixing a liquid product and a solid to form an injecting product and for administering the product, including
- a housing (51) having a gripping portion (57) for gripping it with a hand,
- a receiving means (30), wherein the housing (51) is in threaded engagement with the receiving means (30),
- a two-chamber ampoule (40) which is accommodated by the receiving means (30), which in particular is introduced into the receiving means (30) from the distal end,
wherein the two-chamber ampoule (40) has a bypass (47), a first plunger (45) and a second plunger (46), wherein the two plungers (45, 46) bear sealingly against a hollow-cylindrical housing portion (48) of the two-chamber ampoule (40) and can be displaced along the housing portion (48), wherein the two-chamber ampoule (40) has the bypass (47) in the region of the housing portion (48) and forms an outward bulge and the bypass (47) is axially longer than the first plunger (45), and
- an outer sleeve (29) having a gripping portion for gripping with another hand or an outer sleeve (29) in the form of a gripping portion for gripping with another hand,
wherein the outer sleeve (29) extends over a part of the length of the receiving means (30) and/or the two-chamber ampoule (40) and the housing (51) is arranged between the outer sleeve (29) and the receiving means (30) and is moveable relative to the receiving means (30) into the outer sleeve (29),
wherein the movement is a combined axial movement and rotary movement and by said movement the liquid product and the solid are mixed in the two-chamber ampoule (40) to give the injecting product, wherein for mixing purposes a pressure is exerted on the second plunger (46), which is transmitted by way of the liquid product between the first plunger (45) and the second plunger (46) to the first plunger (45), wherein the second plunger (46) is displaced in the axial direction to the first plunger (45) into the region of the bypass (47), wherein when the first plunger (45) is completely in the region of the bypass (47) the liquid product flows past the first plunger (45) through the bulge of the bypass (47) and mixes with a solid arranged distally from the first plunger (45) to give the injecting product.

2. A device according to the preceding claim **characterised in that** the receiving means (30) and the outer sleeve (29) are non-rotatably and axially fixedly connected.

3. A device according to one of the preceding claims **characterised in that** formed between the outer sleeve (29) and the receiving means (30) is an annular gap into which the at least one part of the housing (51) is moveable.

4. A device according to one of the preceding claims **characterised in that** accommodated by the housing (51) is a plunger rod (52) which at least in a part of the movement of the housing (51) into the outer sleeve (29), in particular in a mixing operation of the two-chamber ampoule (40), is axially fixed relative to the housing (51).

5. A device according to one of the preceding claims **characterised in that** accommodated by the housing (51) is a plunger rod (52) which at least in a part of the movement of the housing (51) into the outer sleeve (29) is axially moveable relative to the housing (51), wherein in particular the plunger rod (52) protrudes proximally from the housing (51).

6. A device according to one of the preceding claims **characterised in that** in a mixed state of the two-chamber ampoule (40) the plunger rod (52) projects proximally out of the housing (51), the plunger rod (52) being displaceable relative to the housing (51) in the distal direction.

7. A device according to one of the preceding claims **characterised in that** the plunger rod (52) has an engagement portion which extends over a part of its length and into which the housing (51) or an element fixed with respect to the housing engages and in particular permits only a limited axial displaceability of the plunger rod (52) relative to the housing (51).

8. A device according to one of the preceding claims **characterised in that** the housing (51) has at least one projection (57) which is arranged at its outer periphery and which in particular is wing-shaped and extends with its narrow side in the axial direction.

9. A device according to one of the preceding claims **characterised in that** the housing (51) has a cylindrical smooth surface.

10. A device according to one of the preceding claims **characterised in that** the housing (51) has at least one information portion which cannot be seen upon the movement of the housing (51) into the outer sleeve (29), for example it disappears under the outer sleeve (29), or for example can be seen through a window in the outer sleeve (29).

11. A device according to one of the preceding claims **characterised in that** the receiving means (30) has a latching element (36) which in at least one position of the housing (51) in relation to the receiving means (30) interacts with at least one counterpart latching member (54, 55).

## Revendications

1. Dispositif de mélange d'un produit liquide et d'une matière solide en un produit d'injection et pour l'administration du produit, comprenant
- un boîtier (51) avec une section de préhension (57) pour la préhension avec une main,
- un logement (30), le boîtier (51) étant en prise filetée avec le logement (30),
- une ampoule à deux chambres (40) qui est reçue par le logement (30), qui est introduite en particulier depuis le côté avant distal dans le logement (30),
dans lequel l'ampoule à deux chambres (40) présente une dérivation (47), un premier (45) et un second piston (46), les deux pistons (45, 46) reposant de manière étanche contre une section de boîtier (48) cylindrique et creuse de l'ampoule à deux chambres (40) et pouvant être déplacés le long de la section de boîtier (48), dans lequel l'ampoule à deux chambres (40) présente la dérivation (47) dans la zone de la section de boîtier (48) et forme un bombement vers l'extérieur, et la dérivation (47) est plus longue axialement que le premier piston (45),
et
- une enveloppe extérieure (29) avec une section de préhension pour la préhension avec une autre main ou une autre enveloppe (29) qui est réalisée comme section de préhension pour la préhension avec une autre main, dans lequel l'enveloppe extérieure (29) s'étend sur une partie de longueur du logement (30) et/ou de l'ampoule à deux chambres (40) et le boîtier (51) est agencé entre l'enveloppe extérieure (29) et le logement (30) et est mobile par rapport au logement (30) dans l'enveloppe extérieure (29),
dans lequel le mouvement est une combinaison d'un mouvement axial et d'un mouvement rotatif et par ce mouvement le produit liquide et le solide sont mélangés dans l'ampoule à deux chambres (40) pour former le produit d'injection, dans lequel pour le mélange une pression est exercée sur le second piston (46) qui est transmise par le produit liquide entre le premier piston (45) et le second piston (46) au premier piston (45), dans lequel le second piston (46) est déplacé dans le sens distal jusqu'à ce que le premier piston (45) soit déplacé dans la zone de la dérivation (47), sachant que lorsque le premier piston (45) se trouve complètement dans la zone de la dérivation (47), le produit liquide s'écoule par le bombement de la dérivation (47) le long du premier piston (45) et se mélange avec un solide agencé distalement par rapport au premier piston (45) pour former le produit d'injection.

2. Dispositif selon l'une des revendications précédentes, **caractérisé en ce que** le logement (30) et l'enveloppe extérieure (29) sont reliés sans pouvoir tourner et de manière fixe axialement.

3. Dispositif selon l'une des revendications précédentes, **caractérisé en ce qu'**une fente annulaire est formée entre l'enveloppe extérieure (29) et le logement (30), dans laquelle fente l'au moins une partie du boîtier (51) est mobile.

4. Dispositif selon l'une des revendications précédentes, **caractérisé en ce qu'**une tige de piston (52) est reçue par le boîtier (51), laquelle est fixe axialement au moins pour une partie du mouvement du boîtier (51) dans l'enveloppe extérieure (29), en particulier pour une opération de mélange de l'ampoule à deux chambres (40), par rapport au boîtier (51).

5. Dispositif selon l'une des revendications précédentes, **caractérisé en ce qu'**une tige de piston (52) est reçue par le boîtier (51), laquelle est mobile axialement au moins pour une partie du mouvement du boîtier (51) dans l'enveloppe extérieure (29) par rapport au boîtier (51), dans lequel en particulier la tige de piston (52) sort de manière proximale du boîtier (52).

6. Dispositif selon l'une des revendications précédentes, **caractérisé en ce que** la tige de piston (52) dépasse dans un état mélangé de l'ampoule à deux chambres (40) de manière proximale du boîtier (51), dans lequel la tige de piston (52) est mobile par rapport au boîtier (51) dans le sens distal.

7. Dispositif selon l'une des revendications précédentes, **caractérisé en ce que** la tige de piston (52) présente une section de mise en prise s'étendant sur une partie de sa longueur dans laquelle le boîtier (51) ou un élément fixé au boîtier se met en prise et permet en particulier seulement une mobilité axiale limitée de la tige de piston (52) par rapport au boîtier (51).

8. Dispositif selon l'une des revendications précédentes, **caractérisé en ce que** le boîtier (51) présente au moins une saillie (57) agencée sur sa périphérie extérieure qui est en particulier en forme d'aile et s'étend avec son côté étroit dans le sens axial.

9. Dispositif selon l'une des revendications précédentes, **caractérisé en ce que** le boîtier (51) présente une surface lisse, cylindrique.

10. Dispositif selon l'une des revendications précédentes, **caractérisé en ce que** le boîtier (51) présente au moins une section d'information qui n'est pas visible lors du mouvement du boîtier (51) dans l'enveloppe extérieure (29), par exemple disparaît sous l'enveloppe extérieure (29) ou est visible par exemple par une fenêtre de l'enveloppe extérieure (29).

11. Dispositif selon l'une des revendications précédentes, **caractérisé en ce que** le logement (30) présente un élément d'encliquetage (36) qui interagit dans au moins une position du boîtier (51) par rapport au logement (30) avec au moins un organe antagoniste d'encliquetage (54, 55).
